# EUROPÄISCHE PATENTSCHRIFT

(11) **EP 4 277 597 B1**
(45) Veröffentlichungstag und Bekanntmachung des Hinweises auf die Patenterteilung: **12.03.2025**
(21) Anmeldenummer: 22701560.9
(22) Anmeldetag: 14.01.2022
(51) Int. Cl.: A61K 9/00, A61K 9/70, A61K 47/14

(54) **ORALER DÜNNFILM**
ORAL THIN FILM
FILM ORODISPERSIBLE

(30) Priorität: 15.01.2021 DE 102021100752
(43) Veröffentlichungstag der Anmeldung: 22.11.2023
(73) Patentinhaber: LTS LOHMANN Therapie-Systeme AG, 56626 Andernach (DE)
(72) Erfinder: SCHMITZ, Christoph, 56598 Rheinbrohl (DE); BAUER, Marius, 56626 Andernach (DE); LINN, Michael, 55596 Waldböckelheim (DE)
(74) Vertreter: Meissner Bolte Partnerschaft mbB
(86) Internationale Anmeldenummer: PCT/EP2022/050786
(87) Internationale Veröffentlichungsnummer: WO 2022/152874

(56) Entgegenhaltungen:
- PRAJAPATI VIPUL D. ET AL: "Pullulan based oral thin film formulation of zolmitriptan: Development and optimization using factorial design", INTERNATIONAL JOURNAL OF BIOLOGICAL MACROMOLECULES, vol. 107, 1 February 2018 (2018-02-01), NL, pages 2075 - 2085, XP055912413, ISSN: 0141-8130, Retrieved from the Internet <URL:https://www.sciencedirect.com/science/article/pii/S0141813017338114/pdfft?md5=8c7dd51a1721bdd1f7f264f7aa234d3a&pid=1-s2.0-S0141813017338114-main.pdf> DOI: 10.1016/j.ijbiomac.2017.10.082
- MASEK JOSEF ET AL: "Multi-layered nanofibrous mucoadhesive films for buccal and sublingual administration of drug-delivery and vaccination nanoparticles - important step towards effective mucosal vaccines", JOURNAL OF CONTROLLED RELEASE, ELSEVIER, AMSTERDAM, NL, vol. 249, 25 July 2016 (2016-07-25), pages 183 - 195, XP029927819, ISSN: 0168-3659, DOI: 10.1016/J.JCONREL.2016.07.036
- JAVIER O MORALES ET AL: "Manufacture and characterization of mucoadhesive buccal films", EUROPEAN JOURNAL OF PHARMACEUTICS AND BIOPHARMACEUTICS, ELSEVIER SCIENCE PUBLISHERS B.V., AMSTERDAM, NL, vol. 77, no. 2, 29 November 2010 (2010-11-29), pages 187 - 199, XP028132513, ISSN: 0939-6411, [retrieved on 20101203], DOI: 10.1016/J.EJPB.2010.11.023

## Beschreibung

Die vorliegende Erfindung betrifft einen oralen Dünnfilm, ein Verfahren zu dessen Herstellung sowie die Verwendung eines solchen oralen Dünnfilms als Arzneimittel.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne, ein-oder mehrschichtige, wirkstoffhaltige Filme auf Polymerbasis, die, wenn sie auf die Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Prajapati Vipul D. at al.: "Pullulan based oral thin film formulation of zolmitriptan: Development and optimization using factorial design", International Journal of Biological Macromolecules, Bd. 107, 1. Februar 2018 (2018-02-01), Seiten 2075-2085 untersucht die Formulierung und Charakterisierung von Pullulan-basierten oralen Dünnfilmen (OTF) von Zolmitriptan mittels Lösungsmittel-Gießverfahren.

WO 2009/124096 A1 offenbart ein Pflaster, ein System und ein Verfahren zur Verabreichung einer durchlässigen Zusammensetzung in ein Subjekt.

Josef Mašek et al.: "Multi-layered nanofibrous mucoadhesive films for buccal and sublingual administration of drug-delivery and vaccination nanoparticies - important step towards effective mucosal vaccines", Journal of controlled release, Elsevier, Bd. 249, 25. Juli 2016, Seiten 183-195, untersucht mukoadhäsive Filme auf der Basis von Nanofasern für die oromukosale Verabreichung von Medikamenten und Impfstoffen.

Orale Dünnfilme in Form eines Schaums können prinzipiell eine hohe Beladung an pharmazeutisch aktivem Wirkstoff aufweisen. Diese hochbeladenen oralen Dünnfilme in Form eines Schaums zeigen in der Regel eine sehr schnelle Zerfallszeit, was für viele Anwendungen vorteilhaft ist.

In anderen Fällen kann es jedoch erwünscht sein, dass die hochbeladenen oralen Dünnfilme in Form eines Schaums länger in der Mundhöhle verbleiben, um z. B. den pharmazeutisch aktiven Wirkstoff für eine transmukosale Aufnahme länger bereitzustellen.

Ein typisches Problem bei der Herstellung von den pharmazeutisch aktiven Wirkstoff langsam freisetzenden oralen Dünnfilmen ist es, dass die dafür typischerweise verwendeten Polymere hochviskose wässrige Lösungen bilden und dadurch des Feststoffanteil einer Wirkstofflösung verringern, was u. a. die Trocknungszeit deutlich verlängert.

Die Aufgabe der vorliegenden Erfindung war es einen oralen Dünnfilm, umfassend eine Polymermatrix in Form eines Hohlräume aufweisenden verfestigten Schaums, bereitzustellen, dessen Zerfallsgeschwindigkeit möglichst breit gesteuert werden kann. Zudem soll der orale Dünnfilm möglichst einfach und möglichst schnell herzustellen sein. Insbesondere sollen hochviskose Lösungen und lange Trocknungszeiten verhindert werden.

Diese Aufgabe wurde überraschenderweise durch einen oralen Dünnfilm gemäß Anspruch 1 gelöst. Ein solcher oraler Dünnfilm ist dadurch gekennzeichnet, dass er eine Polymermatrix in Form eines Hohlräume aufweisenden verfestigten Schaums, wobei die Polymermatrix mindestens einen pharmazeutisch aktiven Wirkstoff, mindestens ein erstes Polymer, das ein wasserlösliches Polymer umfasst, und mindestens ein zweites Polymer, das ein mukoadhäsives Polymer mit einem zahlengemittelten Molekulargewicht von gleich oder größer 100.000 g/mol umfasst, enthält.

Durch den Zusatz eines mukoadhäsiven Polymers mit einem zahlengemittelten Molekulargewicht von gleich oder größer 100.000 g/mol kann eine Schaumformulierung bereitgestellt werden, die eine längere Verweildauer in der Mundhöhle besitzt. Zudem wird durch den Zusatz von einem mukoadhäsiven Polymer mit einem zahlengemittelten Molekulargewicht von gleich oder größer 100.000 g/mol eine erhöhte Mukoadhäsivität erreicht, sodass der Schaum besser an der Mukosa haftet und den Wirkstoff am Resorptionsort halten kann.

Der orale Dünnfilm lässt sich prinzipiell wie die bisher bekannten oralen Dünnfilme herstellen, da das zugesetzte mukoadhäsive Polymer mit einem zahlengemittelten Molekulargewicht von gleich oder größer 100.000 g/mol nur geringen Einfluss auf das physikalische Verhalten beim Aufschlämmen und Trocknen hat. Da bereits bei einer sehr geringen Menge an mukoadhäsiven Polymer mit einem zahlengemittelten Molekulargewicht von gleich oder größer 100.000 g/mol eine zeitverzögerte Auflösung eintreten kann, ist die mögliche Wirkstoffbeladung praktisch nicht beeinträchtigt.

In der vorliegenden Schrift kann "umfassend" auch "bestehend aus" bedeuten.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Der Mukoadhäsionsprozess wird hauptsächlich durch Eigenschaften der verwendeten Polymere, wie Vernetzungsgrad, Kettenlänge und verschiedene funktionelle Gruppen in der Polymerstruktur, beeinflusst. Mukoadhäsive Systeme werden in vielen schleimhautbedeckten Organellen zur Abgabe von Wirkstoffen mit lokaler oder systemischer Wirkung eingesetzt.

Mukoadhäsion kann definiert werden als ein Phänomen der molekularen Anziehungskräfte an den Grenzflächen zwischen den Oberflächen der Mukosa, hier der Mundschleimhaut, und den mukoadhäsiven Polymeren, das es den Polymeren ermöglicht, über einen längeren Zeitraum an der Mukosa zu haften.

Das zweite Polymer, das mindestens ein mukoadhäsives Polymer umfasst, weist ein zahlengemitteltes Molekulargewicht von gleich oder größer 100.000 g/mol, vorzugsweise von größer als 200.000 g/mol oder von größer als 300.000 g/mol oder von größer als 400.000 g/mol oder von größer als 500.000 g/mol oder von größer als 600.000 g/mol oder von größer als 700.000 g/mol oder von größer als 800.000 g/mol oder von größer als 900.000 g/mol auf.

Besonders bevorzugt sind auch mukoadhäsive Polymere mit einem zahlengemittelten Molekulargewicht von gleich oder größer 1.000.000 g/mol oder 2.000.000 g/mol oder 3.000.000 g/mol oder 4.000.000 g/mol oder 5.000.000 g/mol oder 6.000.000 g/mol oder 7.000.000 g/mol.

Das Molekulargewicht wird gemäß dem Fachmann bekannten Methoden, wie beispielsweise Gelpermeationschromatographie, bestimmt.

Vorzugsweise ist das zweite Polymer, das mindestens ein mukoadhäsive Polymer mit einem zahlengemittelten Molekulargewicht von gleich oder größer 100.000 g/mol umfasst, ein wasserlösliches Polymer, wie vorstehend definiert.

Der erfindungsgemäße orale Dünnfilm ist ferner vorzugsweise dadurch gekennzeichnet, dass das erste Polymer ein Polymer mit einem zahlengemittelten Molekulargewicht von kleiner als 100.000 g/mol, vorzugsweise kleiner als 80.000 oder kleiner als 60.000 umfasst.

Das Molekulargewicht wird gemäß dem Fachmann bekannten Methoden, wie beispielsweise Gelpermeationschromatographie, bestimmt.

Das erste Polymer, das ein wasserlösliches Polymer umfasst, ist vorzugsweise ausgewählt aus der Gruppe umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylate, Polyvinylpyrrolidone, Vinylpyrrolidon/Vinylacetat-Copolymere, Polyvinylalkohole, Polyethylenoxidpolymere, Polyacrylamide, Polyethylenglycole, Gelatine, Collagen, Alginate, Pektin, Pullulan, Traganth, Chitosan, Alginsäure, Arabinogalaktan, Galaktomannan, Agar, Agarose, Carrageen und/oder natürlichen Gummen, wobei Polyvinylalkohole besonders bevorzugt sind.

Der erfindungsgemäße orale Dünnfilm ist ferner vorzugsweise dadurch gekennzeichnet, dass das zweite Polymer, das ein mukoadhäsives Polymer mit einem zahlengemittelten Molekulargewicht von gleich oder größer 100.000 g/mol umfasst, ausgewählt ist aus der Gruppe bestehend aus Poly(ethylen)oxidpolymere, Stärkederivaten, Polyacrysäuren, Amylopektinen und/oder Polyvinylalkoholen, insbesondere Mischungen aus Polyacrylaten bzw. Polyacrylsäuren und Amylopektinen.

Geeignete zweite Polymere sind beispielsweise Poly(ethylen)oxidpolymere, wie Polyethylenglykole, mit einem zahlengemittelten Molekulargewicht von etwa 1.000.000 g/mol oder etwa 7.000.000 g/mol, die beispielsweise unter dem Handelsnamen "Polyox WSR N12" oder "Polyox WSR 303" erhältlich sind.

Andere geeignete zweite Polymere sind Mischungen aus Polyacrylat bzw. Polyacrylsäuren und Amylopektin, die unter dem Handelsnamen "Proloc 15" erhältlich sind.

Es sind ferner langkettige Polyvinylalkohole als zweites Polymer geeignet. Beispielsweise ein Polyvinylalkohol mit einem zahlengemittelten Molekulargewicht von etwa 200.000 g/mol, der beispielsweise unter dem Handelsnamen "PVA 40-88" erhältlich ist.

Der erfindungsgemäße orale Dünnfilm ist ferner vorzugsweise dadurch gekennzeichnet, dass das erste Polymer in einer Menge von 40 bis 95 Gew.-%, vorzugsweise von 45 bis 85 und besonders bevorzugt von 48 bis 80, bezogen auf das Gesamtgewicht des oralen Dünnfilms, in dem oralen Dünnfilm enthalten ist.

Vorzugsweise liegt das zweite Polymer in einer Menge von 0,1 bis 20 Gew.-%, vorzugsweise von 2. bis 18 und besonders bevorzugt von 5 bis 15 bezogen auf das Gesamtgewicht des oralen Dünnfilms, in dem oralen Dünnfilm vor.

Der mindestens eine pharmazeutisch aktive Wirkstoff unterliegt prinzipiell keiner Beschränkung, ist aber vorzugsweise ausgewählt aus allen pharmazeutisch aktiven Wirkstoffen, die zur oralen und/oder transmukosalen Applikation geeignet sind.

Bevorzugt sind Wirkstoffe ausgewählt aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin.

Unter Ketamin wird (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Besonders bevorzugt liegt (S)-Ketamin als einziges Stereoisomer von Ketamin vor, da die analgetische und anästhetische Potenz von (S)-Ketamin etwa dreifach höher als die der (R)-Form ist.

Der Wirkstoffgehalt in dem oralen Dünnfilm kann innerhalb relativ weiten Grenzen variieren. Als geeignet kann ein Bereich von 10 bis 60 Gew.-%, bezogen auf das Gesamtgewicht (Trockengesamtgewicht) des oralen Dünnfilms, angegeben werden. In einer Ausführungsform liegt der Anteil an Wirkstoff in dem oralen Dünnfilm eher im unteren Bereich, z.B. wenn der Wirkstoff einen stark unangenehmen Geschmack aufweist, der mit einer größeren Menge an geschmacksmaskierenden Mitteln kompensiert werden muss. In diesem Fall kann als geeigneter Wirkstoffanteil ein Bereich von 10 bis 40 Gew.-% angegeben werden. In einer anderen Ausführungsform liegt der Anteil an Wirkstoff in der erfindungsgemäßen Darreichungsform eher im oberen Bereich, wobei ein Gehalt von 40 bis 60 Gew.-% und insbesondere ein Gehalt von 45 bis 55 Gew.-% als besonders bevorzugt angegeben werden kann.

Der Wirkstoffgehalt pro Dosiereinheit beträgt bis zu 100 mg, vorzugsweise bis zu 50 mg, besonders bevorzugt bis zu 40 mg und meist bevorzugt bis zu 35 mg. Auf der anderen Seite sollte der Mindestwirkstoffgehalt pro Dosiereinheit vorzugsweise 5 mg, weiter bevorzugt 10 mg und meist bevorzugt 15 mg betragen. Je nach Anwendungsfall kann die Wirkstoffgehalt auch im oberen Bereich der vorstehenden Angaben liegen, beispielsweise im Bereich von 50 bis 100 mg. Auch ein Werkstoffgehalt von 30 bis 50 mg kann geeignet sein.

Der erfindungsgemäße orale Dünnfilm ist ferner vorzugsweise dadurch gekennzeichnet, dass der orale Dünnfilm mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder 0,1 bis 5 Gew.-%, bezogen auf das Gesamtgewicht des oralen Dünnfilms, enthalten.

Der erfindungsgemäße mehrschichtige orale Dünnfilm ist prinzipiell in der Anzahl der enthaltenen Schichten nicht beschränkt.

So sind Ausführungsformen denkbar, bei denen der erfindungsgemäße orale Dünnfilm mehrere wirkstoffhaltige Schichten aufweist.

Der erfindungsgemäße orale Dünnfilm ist dadurch gekennzeichnet, dass er in Form eines Hohlräume aufweisenden verfestigten Schaums vorliegt.

Ein Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Matrixschicht. Auf diese Weise wird der vorteilhafte Effekt, die Lösung des oralen Dünnfilms zu beschleunigen, in günstiger Weise beeinflusst.

Ferner können dem oralen Dünnfilm zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften des erfindungsgemäßen oralen Dünnfilms beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 60 µm liegen. Besonders bevorzugt liegt der Durchmesser im Bereich von 10 und 50 µm.

Der erfindungsgemäße orale Dünnfilm besitzt vorzugsweise eine Fläche von 0,5 cm² bis 10 cm², besonders bevorzugt von 2 cm² bis 8 cm².

Das Flächengewicht des erfindungsgemäßen oralen Dünnfilms beträgt vorzugsweise mindestens 10 g/m², bevorzugter mindestens 20 g/m² oder mindestens 30 g/m² oder am meisten bevorzugt mindestens 50 g/m² oder ist kleiner als oder gleich 400 g/m², bevorzugter kleiner als oder gleich 350 g/m² oder kleiner als oder gleich 300 g/m² oder am meisten bevorzugt kleiner als oder gleich 150 g/m². Vorzugsweise beträgt das Flächengewicht 10 bis 400 g/m², bevorzugter 20 bis 350 g/m² oder 30 bis 300 g/m² und am meisten bevorzugt 50 bis 200 g/m².

Bevorzugt weist der erfindungsgemäße orale Dünnfilm eine Dicke von etwa 10 µm bis etwa 1000 µm, besonders bevorzugt von etwa 20 µm bis etwa 500 µm, auf.

Der aus dem oralen Dünnfilm freigesetzte pharmazeutisch aktive Wirkstoff wird vorzugsweise entweder am Applikationsort resorbiert, also über die Mundschleimhaut, oder er wird weitertransportiert und an einem anderen Ort resorbiert (z. B. nach Verschlucken des in der Mundhöhle freigesetzten Wirkstoffs im Magen-Darm-Trakt).

Die Verweildauer des erfindungsgemäßen oralen Dünnfilms am Applikationsort (z. B. Mundraum) bzw. die Zerfallszeit liegt vorzugsweise im Bereich von 10 Sekunden bis 10 Minuten, stärker bevorzugt im Bereich von 30 Sekunden bis 8 Minuten und am meisten bevorzugt im Bereich von 1 Minute bis 5 Minuten.

Die vorliegende Erfindung betrifft auch ein Verfahren zur Herstellung eines oralen Dünnfilms, wie vorstehend beschrieben, umfassend die Schritte:
a) Herstellen einer Lösung, Dispersion oder Schmelze, die mindestens das erste Polymer, das zweite Polymer und den mindestens einen pharmazeutischen Wirkstoff umfasst;
b) Aufschäumen der Lösung, Dispersion oder Schmelze durch Eintragen eines Gases oder Gasgemisches, durch chemische Gaserzeugung, oder durch Entspannen eines gelösten Gases, gegebenenfalls nach vorherigem Zusatz eines schaumstabilisierenden Mittels;
c) Ausstreichen der aufgeschäumten Lösung, Dispersion oder Schmelze auf eine Beschichtungsunterlage; und
d) Verfestigen der ausgestrichenen Lösung, Dispersion oder Schmelze durch Trocknen und Entzug des Lösungsmittels bzw. durch Abkühlung.

Unter "Trocknen" wird verstanden, dass dem oralen Dünnfilm Lösungsmittel, insbesondere Wasser, entzogen wird. Dabei ist es nicht erforderlich, dass dem oralen Dünnfilm sämtliches Lösungsmittel wie Wasser entzogen wird, sondern es ist vielmehr ausreichend, wenn dem oralen Dünnfilm der wesentliche Anteil an Lösungsmittel entzogen wird, so dass sich der Schaum verfestigt. Der orale Dünnfilm kann nach der Trocknung daher einen Restwasseranteil aufweisen.

Durch Lösungsmittel-Entzug verfestigt sich der Schaum während der Trocknung, wobei die gebildeten Hohlräume eine dauerhafte Struktur erhalten. Orale Dünnfilme mit gewünschten Flächenausmaßen oder geometrischen Formen können dadurch erhalten werden, dass die geschäumte Masse vor der Trocknung in entsprechende Formen gegossen wird, oder indem die einzelnen oralen Dünnfilme aus einem größeren Flächenstück ausgestanzt werden.

Die Form, Anzahl und Größe der erzeugten Hohlräume lässt sich mittels verschiedener Verfahrensparameter beeinflussen, z. B. durch die Art und Konzentration der Polymere, durch die Viskosität der Polymermasse, durch die Steuerung des Aufschäumungsvorgangs, durch die Auswahl der schaumstabilisierenden Mittel, etc.

Alternativ zu dem vorstehend beschriebenen Verfahren ist es möglich, den erfindungsgemäßen oralen Dünnfilm über ein Verfahren herzustellen, bei dem die Ausbildung der Hohlräume im Inneren der Polymermatrix durch Einbringen eines hydrophoben, mit dem für die Herstellung der genannten Lösung oder Dispersion verwendeten Lösungsmittel nicht mischbaren Lösungsmittels erfolgt.

Dabei wird eine Emulsion erzeugt, welche das hydrophobe Lösungsmittel in Form fein verteilter Tröpfchen enthält.

Durch das Entfernen der Lösungsmittel während der nachfolgenden Trocknung bleiben in der Polymermatrix tröpfchen- oder blasenförmige Hohlräume zurück.

Ferner kann in Abwandlung des oben beschriebenen Verfahrens die Erzeugung der genannten Hohlräume auf die Weise geschehen, dass der polymer-und wirkstoffhaltigen Lösung oder Dispersion Hilfsstoffe zugesetzt werden, die ein Gas oder Gase bilden, wodurch die Masse aufgeschäumt wird. Dieses Aufschäumen durch Gasentwicklung kann entweder während der Herstellung der Polymermasse oder während der Beschichtung dieser Masse auf die Unterlage erfolgen, oder erst während des nachfolgenden Trocknungsprozesses. Zur Gasbildung geeignete Stoffe oder Stoffgemische sind dem Fachmann bekannt. Das Aufschäumen kann ferner auch durch Entspannung eines vorher gelösten Gases bewirkt werden. Als Gas kann insbesondere ein inertes Gas, wie Stickstoff, Kohlendioxid oder Helium, oder eine Mischung, davon verwendet werden.

Bei der Herstellung des erfindungsgemäßen oralen Dünnfilms kann alternativ auch von einer Schmelze des Matrixpolymers bzw. Polymergemisches ausgegangen werden. Die Verarbeitung erfolgt grundsätzlich ähnlich wie bei aus dem Stand der Technik bekannten Heißschmelz ("hot melt") - Beschichtungsmassen.

In die genannte Polymerschmelze wird durch eine der vorstehend genannten Methoden Gas oder ein Gasgemisch eingetragen, um ein Aufschäumen der Schmelze zu bewirken. Anschließend wird die Schmelze auf eine geeignete Unterlage ausgestrichen oder extrudiert oder in eine Form gegossen, und dann abkühlen bzw. erstarren lassen. Eine Verarbeitung aus der Schmelze kommt nicht in Frage, wenn der vorgesehene Wirkstoff bei der Schmelztemperatur der Polymerschmelze instabil oder flüchtig ist. Falls erforderlich, können der Polymerschmelze Hilfsstoffe zur Herabsetzung des Schmelzpunktes beigefügt werden.

Gemäß einer weiteren Abwandlung der vorstehend beschriebenen Herstellungsverfahren wird die Polymermatrix zunächst in Form eines Blockes hergestellt. Von diesem wird nachfolgend, d. h. nach erfolgter Trocknung bzw. Erstarrung, durch Zerschneiden die gewünschte Darreichungsform abgetrennt.

Dies bedeutet, dass in dieser Ausführungsform die vorstehend beschriebenen Schritte c) und d) durch die folgende Schritte c2) und d2) ersetzt werden:
c2) Herstellen eines Blockes, ausgehend von der Lösung, Dispersion oder Schmelze;
d2) Zerschneiden des verfestigten Blocks, um flächenförmige Formen zu erhalten.

Die vorliegende Erfindung betriff auch einen oralen Dünnfilm erhältlich gemäß dem vorstehend beschriebene Verfahren.

Außerdem betrifft die vorliegende Erfindung einen oralen Dünnfilm, wie vorstehend beschrieben oder erhältlich nach dem vorstehend beschriebenen Verfahren, als Arzneimittel.

Die vorliegende Erfindung betrifft zudem einen oralen Dünnfilm, wie vorstehend beschrieben oder erhältlich nach dem vorstehend beschriebenen Verfahren, wobei Ketamin, vorzugsweise (S)-Ketamin, oder ein pharmazeutisch akzeptables Salz davon als pharmezutisch aktiver Wirkstoff vorhanden ist, zur Verwendung in der Behandlung von Schmerzen und/oder Depressionen, insbesondere zur Reduzierung des Suizidrisikos, und/oder zur Verwendung als Allgemeinanästhetikum, vorzugsweise zur Einleitung oder Durchführung einer Vollnarkose oder als Ergänzung bei Regionalanästhesien und/oder als Analgetikum.

Die vorstehend für den mehrschichtigen oralen Dünnfilm aufgeführten bevorzugten Ausführungsformen gelten auch für das erfindungsgemäße Verfahren, den durch dieses Verfahren erhaltenen mehrschichtigen oralen Dünnfilm und dessen Verwendung als Arzneimittel.

Die Erfindung wird nachfolgend anhand von nicht beschränkenden Beispielen näher erläutert.

### Beispiele

In den folgenden Beispielen werden folgende Polymere eingesetzt.

| | |
|---|---|
| PVA 4-88: | Polyvinylalkohol (MW < 100,000) |
| PVA 40-88: | Polyvinylalkohol (MW > 100,000) |
| Polyox WSR N12: | Poly(ethylen)oxidpolymer (MW etwa 1.000.000) |
| Polyox WSR 303: | Poly(ethylen)oxidpolymer (MW etwa 7.000.000) |
| Proloc 15: | Mischung aus Polyacrylat und Amylopektin (MW > 100,000) |

### Beispiel 1

Es wurden geschäumte orale Dünnfilme mit den Zusammensetzungen gemäß den Tabellen 1 bis 5 ohne pharmazeutisch aktiven Wirkstoff wie folgt hergestellt.

Die Herstellung der einzelnen oralen Dünnfilme erfolgte durch dem Fachmann bekannte Techniken der Masseherstellung, z.B. durch Rühren/Mischen der enthaltenen Komponenten mittels Rührmotor und geeigneter Rührwerkzeuge. In die so erhaltene Masse wird durch Rühren Gas eingeschlagen, z.B. mittels einer Schaumaufschlagmaschine. Die schaumige Masse wird durch geeignete Geräte (Walzenauftragswerk, Rakel, Streichkasten, etc) in einer konstanten Schichtdicke auf einen Beschichtungsträger beschichtet. Als Beschichtungsträger können alle temperaturstabilen bahnförmigen Materialien dienen, von denen sich der trockene Film wieder entfernen lässt. Dies kann durch die Materialauswahl des Beschichtungsträgers gewährleistet werden (unterschiedliche Oberflächenspannungen zwischen schaumförmiger Masse und Substrat), oder durch geeignete dehäsive Beschichtungen des Beschichtungsträgers mit z.B. Silikonen oder Fluorpolymeren. Das oder die enthaltenen Prozesslösemittel, im Regelfall Wasser oder Mischungen aus Wasser und organischen, wassermischbaren Lösemitteln, werden durch Trocknen entfernt. Aus der so erhaltenen, festen Schaumschicht lassen sich die oralen Dünnfilme durch Schneiden oder Stanzen in der entsprechenden Größe konfektionieren.

**Tabelle 1:**

| Inhaltsstoff | Funktion | Menge [Gew.-%] |
|---|---|---|
| PVA 4-88 | Polymer | 74,6 |
| Polyox WSR N12 | Polymer (Zerfallsverzögerer) | 2,2 |
| FD&C Red 40 | Farbstoff | 0,4 |
| Saccharin Na | Süßungsmittel | 2,0 |
| Sucralose | Süßungsmittel | 4,0 |
| Glycerin | Weichmacher | 9,0 |
| Menthol | Aroma | 2,0 |
| Cherry Flavor | Aroma | 6,0 |

**Tabelle 2:**

| Inhaltsstoff | Funktion | Menge [Gew.-%] |
|---|---|---|
| PVA 4-88 | Polymer | 81,1 |
| Polyox WSR 303 | Polymer (Zerfallsverzögerer) | 0,5 |
| FD&C Red 40 | Farbstoff | 0,4 |
| Saccharin Na | Süßungsmittel | 2,0 |
| Sucralose | Süßungsmittel | 4,0 |
| Glycerin | Weichmacher | 9,0 |
| Cherry Flavor | Aroma | 6,0 |

**Tabelle 3:**

| Inhaltsstoff | Funktion | Menge [Gew.-%] |
|---|---|---|
| PVA 4-88 | Polymer | 80,6 |
| Polyox WSR 3032 | Polymer (Zerfallsverzögerer) | 1,0 |
| FD&C Red 40 | Farbstoff | 0,4 |
| Saccharin Na | Süßungsmittel | 2,0 |
| Sucralose | Süßungsmittel | 4,0 |
| Glycerin | Weichmacher | 9,0 |
| Menthol | Aroma | 2,0 |
| Cherry Flavor | Aroma | 6,0 |

**Tabelle 4:**

| Inhaltsstoff | Funktion | Menge [Gew.-%] |
|---|---|---|
| PVA 4-88 | Polymer | 79,6 |
| Polyox WSR 3032 | Polymer (Zerfallsverzögerer) | 2,0 |
| FD&C Red 40 | Farbstoff | 0,4 |
| Saccharin Na | Süßungsmittel | 2,0 |
| Sucralose | Süßungsmittel | 4,0 |
| Glycerin | Weichmacher | 9,0 |
| Menthol | Aroma | 2,0 |
| Cherry Flavor | Aroma | 6,0 |

**Tabelle 5:**

| Inhaltsstoff | Funktion | Menge [Gew.-%] |
|---|---|---|
| PVA 4-88 | Polymer | 79,6 |
| Proloc 15 | Polymer (Zerfallsverzögerer) | 2,0 |
| FD&C Red 40 | Farbstoff | 0,4 |
| Saccharin Na | Süßungsmittel | 2,0 |
| Sucralose | Süßungsmittel | 4,0 |
| Glycerin | Weichmacher | 9,0 |
| Menthol | Aroma | 2,0 |
| Cherry Flavor | Aroma | 6,0 |

Die oralen Dünnfilme mit den Zusammensetzungen gemäß Tabelle 1 bis 6 wiesen deutlich längerer Zerfallszeiten auf als Zusammensetzungen die kein Polymer mit einem MW von größer als 100.000 g/mol enthielten.

### Beispiel 2

Es wurden geschäumte orale Dünnfilme mit den Zusammensetzungen gemäß den Tabellen 6 und 7 mit (S)-Ketamin HCl als aktivem Wirkstoff wie oben ausgeführt, hergestellt.

**Tabelle 6:**

| Inhaltsstoff | Funktion | Menge per Dosis [m/1,67 cm³) | Menge [Gew.-%] |
|---|---|---|---|
| (S)-Ketamin HCl | Wirkstoff | 16,1 | 55,00 |
| PVA 4-88 | Polymer | 5,1 | 17,25 |
| PVA 40-88 | Polymer (Zerfallsverzögerer) | 5,1 | 17,25 |
| Saccarin Na | Süßungsmittel | 0,3 | 1,00 |
| Sucralose | Süßungsmittel | 0,6 | 2,00 |
| Gylcerol | Weichmacher | 1,3 | 4,50 |
| Geschmacksstoff PHL-131985 | Aroma | 0,3 | 1,00 |
| Geschmacksstoff PHL-097336 | Aroma | 0,6 | 2,00 |
| Zerfallszeit | 31 sec | | |

**Tabelle 7:**

| Inhaltsstoff | Funktion | Menge per Dosis [m/1,67 cm³) | Menge [Gew.-%] |
|---|---|---|---|
| (S)-Ketamin HCl | Wirkstoff | 16,1 | 50,00 |
| PVA 4-88 | Polymer | 12,8 | 39,50 |
| Saccarin Na | Süßungsmittel | 0,3 | 1,00 |
| Sucralose | Süßungsmittel | 0,6 | 2,00 |
| Gylcerol | Weichmacher | 1,4 | 4,50 |
| Cherry Flavor | Aroma | 1,0 | 3,00 |
| Zerfallszeit | etwa 9 sec | | |

Der orale Dünnfilm mit der Zusammensetzung gemäß Tabelle 6 wies eine deutlich längere Zerfallszeit auf als der orale Dünnfilm mit der Zusammensetzung gemäß Tabelle 7, der kein Polymer mit einem MW von größer als 100.000 g/mol enthielt.

Für den geschäumten oralen Dünnfilm mit der Zusammensetzung gemäß Tabelle 6 wurde zudem die Freisetzungsraten des Wirkstoffs bestimmt.

### Freisetzungsmessung:

Bei der in-vitro Freisetzung wird S-Ketamin aus S-Ketamin HCl oralen Dünnfilmen (OTF) freigesetzt und bestimmt. Der Wirkstoff wird in Phosphatpuffer pH 6.8 USP freigesetzt und dann durch eine Gradienten-Umkehrphasen HPLC-Methode bestimmt. Die Quantifizierung erfolgte gegen einen externen Standard.

Die Freisetzung wird mit Dissolution Apparatus 2 - (Paddle over sinker) gemäß USP <711> durchgeführt.

**Tabelle 8:**

| Sinker: | Stainless Steel Capsule Sinker with 10 Spirals, 31.0 x 11.0 mm Capacity (Sotax Style) |
|---|---|
| Rührgeschwindigkeit: | 50 rpm |
| Abstand zwischen dem Vesselboden und der Unterkante des Paddle: | 25 mm ± 2 mm |
| Temperatur: | 37°C ± 0.5°C |
| Freisetzungsmedium: | Phosphatpuffer pH 6.8 USP |
| Volumen Freisetzungsmedium: | 900 mL |
| Probennahmezeitpunkte: | 1, 3, 5, 10 und 15 min |
| Probenvolumen: | 5,0 mL |

Die ermittelten Freisetzungsraten sind in Tabelle 9 zusammengefasst.

**Tabelle 9:**

| In-vitro Freisetzung | | [% von LC*] |
|---|---|---|
| nach 1min | Mean | 57 |
| | Min | 52 |
| | Max | 59 |
| | RSD [%] | 5,2 |
| nach 3min | Mean | 101 |
| | Min | 97 |
| | Max | 105 |
| | RSD [%] | 2,8 |
| nach 5min | Mean | 104 |
| | Min | 101 |
| | Max | 110 |
| | RSD [%] | 2,9 |
| nach 10min | Mean | 104 |
| | Min | 102 |
| | Max | 108 |
| | RSD [%] | 2,3 |
| nach 15min | Mean | 104 |
| | Min | 101 |
| | Max | 109 |
| | RSD [%] | 2,7 |

| | | |
|---|---|---|
| *Lable Claim = Sollgehalt | | |

Die Wirkstofffreisetzung aus einem oralen Dünnfilm gemäß Tabelle 7 (fastrelease) wurde mit der Wirkstofffreisetzung aus einem oralen Dünnfilm, der sich durch eine besonders langsamere Wirkstofffreisetzung (slow-release) auszeichnet, verglichen.

Die Zusammensetzung des oralen Dünnfilms mit langsamer Wirkstofffreisetzung ist in Tabelle 10 angegeben.

**Tabelle 10:**

| Inhaltsstoff | Funktion | Menge [Gew.-%] |
|---|---|---|
| (S)-Ketamin HCl | Wirkstoff | 30,00 |
| Proloc 15 | Polymer | 10,00 |
| PVA 40-88 | Polymer (Zerfallsverzögerer) | 49,50 |
| Saccarin Na | Süßungsmittel | 1,00 |
| Sucralose | Süßungsmittel | 2,00 |
| Gylcerol | Weichmacher | 4,50 |
| Cherry Flavor | Aroma | 3,00 |
| Flächengewicht (trocken) | | 152,9 g/m² |
| Flächengewicht (incl. Res. Lösungsmittel) | | 156,0 g/m² |

Für die geschäumten oralen Dünnfilme mit den Zusammensetzungen gemäß Tabelle 7 und Tabelle 10 wurden zudem die Freisetzungsraten des Wirkstoffs, wie oben ausgeführt, bestimmt.

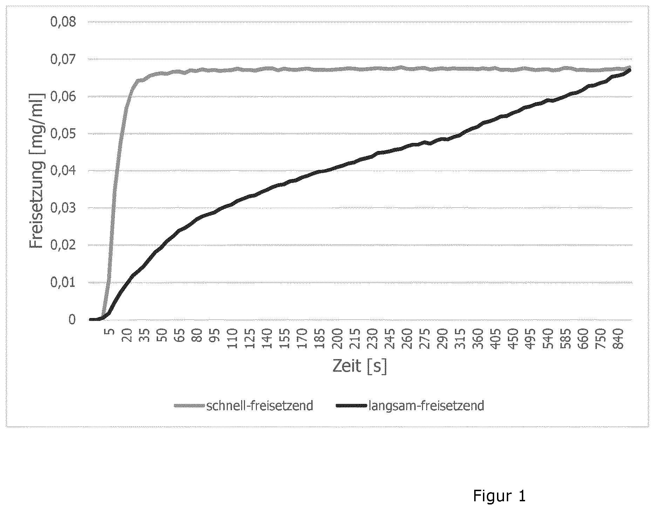

Die ermittelten Freisetzungsraten sind in Figur 1 dargestellt.

### Beispiel 3:

Es wurde ein geschäumter oraler Dünnfilm mit der Zusammensetzung gemäß der Tabelle 11 mit (S)-Ketamin HCl als aktivem Wirkstoff wie oben ausgeführt hergestellt.

**Tabelle 11:**

| Inhaltsstoff | Funktion | Menge per Dosis [mg/2,72 cm³) | Menge [Gew.-%] |
|---|---|---|---|
| (S)-Ketamin HCl | Wirkstoff | 16,1 | 50,00 |
| PVA 4-88 | Polymer | 12,8 | 39,50 |
| Saccarin Na | Süßungsmittel | 0,3 | 1,00 |
| Sucralose | Süßungsmittel | 0,6 | 2,00 |
| Gylcerol | Weichmacher | 1,5 | 4,50 |
| Masking Flavor | Aroma | 0,3 | 1,00 |
| Raspberry Flavor | Aroma | 0,6 | 2,00 |
| Zerfallszeit | etwa 3 sec | | |

Für den geschäumten oralen Dünnfilm mit der Zusammensetzung gemäß Tabelle 11 wurde zudem die Freisetzungsrate des Wirkstoffs, wie oben ausgeführt, bestimmt.

Die ermittelten Freisetzungsraten sind in Tabelle 12 zusammengefasst.

**Tabelle 12:**

| In-vitro Freisetzung | | [% von LC*] |
|---|---|---|
| nach 1min | Mean | 100 |
| | Min | 93 |
| | Max | 106 |
| | RSD [%] | 5,0 |
| nach 3min | Mean | 104 |
| | Min | 100 |
| | Max | 109 |
| | RSD [%] | 3,4 |
| nach 5min | Mean | 105 |
| | Min | 100 |
| | Max | 109 |
| | RSD [%] | 3,6 |
| nach 10min | Mean | 105 |
| | Min | 100 |
| | Max | 110 |
| | RSD [%] | 3,8 |
| nach 15min | Mean | 105 |
| | Min | 101 |
| | Max | 109 |
| | RSD [%] | 3,6 |

| | | |
|---|---|---|
| *Lable Claim = Sollgehalt | | |

## Patentansprüche

1. Oraler Dünnfilm, umfassend eine Polymermatrix in Form eines Hohlräume aufweisenden verfestigten Schaums, wobei die Polymermatrix mindestens einen pharmazeutisch aktiven Wirkstoff, mindestens ein erstes Polymer, das ein wasserlösliches Polymer umfasst, und mindestens ein zweites Polymer, das ein mukoadhäsives Polymer mit einem zahlengemittelten Molekulargewicht von gleich oder größer 100.000 g/mol umfasst, enthält.

2. Oraler Dünnfilm gemäß Anspruch 1, **dadurch gekennzeichnet, dass** das mindestens eine mukoadhäsive Polymer ein wasserlösliches Polymer umfasst.

3. Oraler Dünnfilm gemäß irgendeinem der vorhergehenden Ansprüche, **dadurch gekennzeichnet, dass** das erste Polymer ein Polymer mit einem zahlengemittelten Molekulargewicht von kleiner als 100.000 g/mol umfasst.

4. Oraler Dünnfilm gemäß irgendeinem der vorhergehenden Ansprüche, **dadurch gekennzeichnet, dass** das erste Polymer ausgewählt ist aus der Gruppe umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäure, Polyacrylate, Polyvinylpyrrolidone, Vinylpyrrolidon/Vinylacetat-Copolymere Polyvinylalkohole, Poly(ethylen)oxidpolymere, Polyacrylamide, Polyethylenglycole, Gelatine, Collagen, Alginate, Pektin, Pullulan, Traganth, Chitosan, Alginsäure, Arabinogalaktan, Galaktomannan, Agar, Agarose, Carrageen und/oder natürliche Gummen.

5. Oraler Dünnfilm gemäß irgendeinem der vorhergehenden Ansprüche, **dadurch gekennzeichnet, dass** das zweite Polymer ausgewählt ist aus der Gruppe umfassend aus Poly(ethylen)oxidpolymere, Stärkederivaten, Polyacrysäuren, Amylopektinen und/oder Polyvinylalkoholen, insbesondere Mischungen aus Polyacrylaten bzw. Polyacrylsäuren und Amylopektinen.

6. Oraler Dünnfilm gemäß irgendeinem der vorhergehenden Ansprüche, **dadurch gekennzeichnet, dass** das erste Polymer in einer Menge von 40 bis 95 Gew.-%, bezogen auf das Gesamtgewicht des oralen Dünnfilms, in dem oralen Dünnfilm enthalten ist.

7. Oraler Dünnfilm gemäß irgendeinem der vorhergehenden Ansprüche, **dadurch gekennzeichnet, dass** das zweite Polymer in einer Menge von 0,1 bis 20 Gew.-%, bezogen auf das Gesamtgewicht des oralen Dünnfilms, in dem oralen Dünnfilm enthalten ist.

8. Oraler Dünnfilm gemäß irgendeinem der vorhergehenden Ansprüche, **dadurch gekennzeichnet, dass** der mindestens eine pharmazeutisch aktive Wirkstoff in einer Menge von 10 bis 60 Gew.-%, bezogen auf das Gesamtgewicht des oralen Dünnfilms, in dem oralen Dünnfilm enthalten ist.

9. Oraler Dünnfilm gemäß irgendeinem der vorhergehenden Ansprüche, **dadurch gekennzeichnet, dass** die Hohlräume voneinander isoliert sind und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

10. Oraler Dünnfilm gemäß irgendeinem der vorhergehenden Ansprüche, **dadurch gekennzeichnet, dass** die Hohlräume miteinander in Verbindung stehen und vorzugsweise ein die Polymermatrix durchdringendes Kanalsystem bilden.

11. Oraler Dünnfilm gemäß irgendeinem der vorhergehenden Ansprüche, **dadurch gekennzeichnet, dass** die genannten Hohlräume einen Volumenanteil von 5 bis 98 %, bevorzugt von 50 bis 80 %, bezogen auf das Gesamtvolumen des oralen Dünnfilms, ausmachen.

12. Verfahren zur Herstellung eines oralen Dünnfilms gemäß irgendeinem der Ansprüche 1 bis 11, **gekennzeichnet durch** das
a) Herstellen einer Lösung, Dispersion oder Schmelze, die mindestens das erste Polymer, das zweite Polymer und den mindestens einen pharmazeutischen Wirkstoff umfasst;
b) Aufschäumen der Lösung, Dispersion oder Schmelze durch Eintragen eines Gases oder Gasgemisches, durch chemische Gaserzeugung, oder durch Entspannen eines gelösten Gases, gegebenenfalls nach vorherigem Zusatz eines schaumstabilisierenden Mittels;
c) Ausstreichen der aufgeschäumten Lösung, Dispersion oder Schmelze auf eine Beschichtungsunterlage; und
d) Verfestigen der ausgestrichenen Lösung, Dispersion oder Schmelze durch Trocknen und Entzug des Lösungsmittels bzw. durch Abkühlung.

13. Verfahren gemäß Anspruch 12, **dadurch gekennzeichnet, dass** die Schritte c) und d) durch die folgende Schritte c2) und d2) ersetzt werden:
c2) Herstellen eines Blockes, ausgehend von der Lösung, Dispersion oder Schmelze;
d2) Zerschneiden des verfestigten Blocks, um flächenförmige Formen zu erhalten.

14. Oraler Dünnfilm erhältlich durch ein Verfahren gemäß irgendeinem der Ansprüche 12 oder 13.

15. Oraler Dünnfilm gemäß irgendeinem der Ansprüche 1 bis 12 oder Anspruch 14 zur Verwendung als Arzneimittel.

## Claims

1. An oral thin film comprising a polymer matrix in the form of a solidified foam having voids, wherein the polymer matrix contains at least one active pharmaceutical ingredient, at least one first polymer which comprises a water-soluble polymer and at least one second polymer which comprises a mucoadhesive polymer having a number-average molecular weight of equal to or greater than 100,000 g/mol.

2. The oral thin film according to claim 1, **characterised in that** the at least one mucoadhesive polymer comprises a water-soluble polymer.

3. The oral thin film according to any one of the preceding claims, **characterised in that** the first polymer comprises a polymer having a number-average molecular weight of less than 100,000 g/mol.

4. The oral thin film according to any one of the preceding claims, **characterised in that** the first polymer is selected from the group comprising starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, vinyl pyrrolidone/vinyl acetate copolymers, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

5. The oral thin film according to any one of the preceding claims, **characterised in that** the second polymer is selected from the group comprising polyethylene oxide polymers, starch derivatives, polyacrylic acids, amylopectins and/or polyvinyl alcohols, especially mixtures from polyacrylates or polyacrylic acids and amylopectins.

6. The oral thin film according to any one of the preceding claims, **characterised in that** the first polymer is contained in the oral thin film in an amount of 40 to 95 wt.% in relation to the total weight of the oral thin film.

7. The oral thin film according to any one of the preceding claims, **characterised in that** the second polymer is contained in the oral thin film in an amount of 0.1 to 20 wt.% in relation to the total weight of the oral thin film.

8. The oral thin film according to any one of the preceding claims, **characterised in that** the at least one active pharmaceutical ingredient is contained in the oral thin film in an amount of 10 to 60 wt.% in relation to the total weight of the oral thin film.

9. The oral thin film according to any one of the preceding claims, **characterised in that** the voids are isolated from one another and are preferably present in the form of bubbles, the voids being filled with air or a gas, preferably with an inert gas, especially preferably with nitrogen, carbon dioxide, helium or a mixture of at least two of these gases.

10. The oral thin film according to any one of the preceding claims, **characterised in that** the voids are connected to one another and preferably form a channel system penetrating the polymer matrix.

11. The oral thin film according to any one of the preceding claims, **characterised in that** said voids account for a volume fraction of from 5 to 98%, preferably from 50 to 80%, in relation to the total volume of the oral thin film.

12. A method for producing an oral thin film according to any one of claims 1 to 11, **characterised by** the steps of:
a) producing a solution, dispersion or melt which at least comprises the first polymer, the second polymer and the at least one active pharmaceutical ingredient;
b) foaming the solution, dispersion or melt by introducing a gas or gas mixture, by chemical gas generation, or by expanding a dissolved gas, optionally after prior addition of a foam-stabilising agent;
c) spreading the foamed solution, dispersion or melt onto a coating substrate; and
d) solidifying the spread solution, dispersion or melt by drying and removal of the solvent or by cooling.

13. The method according to claim 12, **characterised in that** steps c) and d) are replaced by the following steps c2) and d2):
c2) producing a block, starting from the solution, dispersion or melt;
d2) cutting the solidified block in order to obtain planar forms.

14. An oral thin film obtainable by a method according to any one of claims 12 or 13.

15. Oral thin film according to any one of claims 1 to 12 or claim 14 for use as a medicament.

## Revendications

1. Film mince oral comprenant une matrice polymère sous la forme d'une mousse solidifiée présentant des cavités, dans lequel la matrice polymère comprend au moins un principe pharmaceutiquement actif, au moins un premier polymère, qui comprend un polymère hydrosoluble, et au moins un deuxième polymère, qui comprend un polymère muco-adhésif avec un poids moléculaire moyen supérieur ou égal à 100 000 g/mol.

2. Film mince oral selon la revendication 1, **caractérisé en ce que** l'au moins un polymère muco-adhésif comprend un polymère hydrosoluble.

3. Film mince oral selon l'une quelconque des revendications précédentes, **caractérisé en ce que** le premier polymère comprend un polymère avec un poids moléculaire moyen en nombre inférieur à 100 000 g/mol.

4. Film mince oral multicouche selon l'une quelconque des revendications précédentes, dans lequel le premier polymère est choisi parmi le groupe comprenant les amidons et les dérivés d'amidon, les dextranes, les dérivés de cellulose, tels que la carboxyméthylcellulose, l'hydroxypropylcellulose, l'hydroxyéthylcellulose, l'hydroxypropylméthylcellulose, l'hydroxypropyléthylcellulose, la carboxyméthylcellulose de sodium, l'éthylcellulose ou la propylcellulose, l'acide polyacrylique, les polyacrylates, les polyvinylpyrrolidones, les copolymères de vinylpyrrolidone/acétate de vinyle, les alcools polyvinyliques, les polymères d'oxyde de (poly)éthylène, les polyacrylamides, les polyéthylènes glycol, la gélatine, le collagène, les alginates, la pectine, le pullulane, la gomme adragante, le chitosan, l'acide alginique, l'arabinogalactane, le galactomannane, la gélose, l'agarose, le carraghénane et les gommes naturelles.

5. Film mince oral selon l'une quelconque des revendications précédentes, **caractérisé en ce que** le deuxième polymère est choisi parmi le groupe comprenant les polymères d'oxyde de poly(éthylène), les dérivés d'amidon, les acides polyacryliques, les amylopectines et/ou les alcools polyvinyliques, en particulier les mélanges de polyacrylates ou d'acides polyacryliques et d'amylopectines.

6. Film mince oral selon l'une quelconque des revendications précédentes, **caractérisé en ce que** le premier polymère est contenu dans le film mince oral en une quantité de 40 à 95 % en poids par rapport au poids total du film mince oral.

7. Film mince oral selon l'une quelconque des revendications précédentes, **caractérisé en ce que** le deuxième polymère est contenu dans le film mince oral en une quantité de 0,1 à 20 % en poids par rapport au poids total du film mince oral.

8. Film mince oral selon l'une quelconque des revendications précédentes, **caractérisé en ce que** l'au moins un principe pharmaceutiquement actif est contenu dans le film mince oral en une quantité de 10 à 60 % en poids par rapport au poids total du film mince oral.

9. Film mince oral selon l'une quelconque des revendications précédentes, **caractérisé en ce que** les cavités sont isolées les unes des autres et se présentent de préférence sous la forme de bulles, dans lequel les cavités sont remplies d'air ou d'un gaz, de préférence d'un gaz inerte, de manière particulièrement préférée d'azote, de dioxyde de carbone, d'hélium ou d'un mélange d'au moins deux desdits gaz.

10. Film mince oral selon l'une quelconque des revendications précédentes, **caractérisé en ce que** les cavités sont reliées entre elles et forment de préférence un système de canaux pénétrant la matrice polymère.

11. Film mince oral selon l'une quelconque des revendications précédentes, **caractérisé en ce que** lesdites cavités représentent un pourcentage volumique de 5 à 98 %, de manière préférée de 50 à 80 %, par rapport au volume total du film mince oral.

12. Procédé de fabrication d'un film mince oral selon l'une quelconque des revendications 1 à 11, **caractérisé par**
a) la fabrication d'une solution, d'une dispersion ou d'une matière fondue, qui comprend au moins le premier polymère, le deuxième polymère et l'au moins un principe pharmaceutiquement actif ;
b) le moussage de la solution, de la dispersion ou de la matière fondue par apport d'un gaz ou d'un mélange de gaz, par production chimique de gaz ou par détente d'un gaz dissous, le cas échéant après ajout préalable d'un agent stabilisant la mousse ;
c) l'étalement de la solution, de la dispersion ou de la matière fondue moussée sur un support de revêtement ; et
d) la solidification de la solution, de la dispersion ou de la matière fondue étalée par séchage et extraction du solvant ou par refroidissement.

13. Procédé selon la revendication 12, **caractérisé en ce que** les étapes c) et d) sont remplacées par les étapes suivantes c2) et d2) :
c2) de fabrication d'un bloc à partir de la solution, de la dispersion ou de la matière fondue ;
d2) de découpage du bloc solidifié pour obtenir des formes planes.

14. Film mince oral pouvant être obtenu par un procédé selon l'une quelconque des revendications 12 ou 13.

15. Film mince oral selon l'une quelconque des revendications 1 à 12 ou selon la revendication 14 destiné à être utilisé comme médicament.
